# EUROPÄISCHE PATENTSCHRIFT

(11) **EP 2 281 516 B1**
(45) Veröffentlichungstag und Bekanntmachung des Hinweises auf die Patenterteilung: **14.12.2011**
(21) Anmeldenummer: 09009773.4
(22) Anmeldetag: 29.07.2009
(51) Int. Cl.: A61B 17/29, A61B 19/00

(54) **Chirurgisches Instrument**
Surgical instrument
Instrument chirurgical

(43) Veröffentlichungstag der Anmeldung: 09.02.2011
(73) Patentinhaber: TMT Tschida Medizin Technik, 78580 Bärenthal (DE)
(72) Erfinder: Tschida, Peter, 78580 Bärenthal (DE)
(74) Vertreter: Späth, Dieter

(56) Entgegenhaltungen:
- WO-A-2005/009255
- DE-A1- 19 501 258
- US-A1- 2001 056 283

## Beschreibung

In dieser Beschreibung werden die Begriffe "distal" und "proximal" mit Bezug auf den Patienten gebraucht.

Die Offenlegungsschrift DE 42 38 619 A1 offenbart ein chirurgisches Instrument mit einer Hülle und einer Seele, die in der Hülle angeordnet und zur Betätigung des Instruments gegenüber der Hülle verschiebbar ist. Seele bedeutet ein in der Hülle verschiebbares Element zur Übertragung einer Zug- und/oder Druckkraft. Die Hülle des bekannten chirurgischen Instruments ist ein Rohr, die Seele eine Stange. An einem proximalen Ende weist das bekannte chirurgische Element ein Maul mit zwei gegeneinander schwenkbaren Maulschenkeln auf, die durch Verschieben der Seele gegenüber der Hülle geschwenkt werden, um das Maul zu öffnen oder zu schließen. Das Maul kann beispielsweise eine Zange oder eine Schere sein. Es können beide Maulschenkel schwenkbar oder ein Maulschenkel feststehend und der andere Maulschenkel schwenkbar sein. An einem distalen Ende weist das bekannte chirurgische Instrument Zangengriffe auf, mittels derer sich die Seele gegenüber der Hülle verschieben und das Maul schließen und öffnen, d.h. das Instrument sich betätigen lässt.

Das bekannte chirurgische Instrument ist, wie an sich alle chirurgischen Instrumente, ein feinmechanisches Werkzeug mit beweglichen Teilen, die auf engstem Raum an Enden der Hülle und in der Hülle angeordnet und deswegen schwierig zu reinigen sind. Eine gute Reinigung ist für chirurgische Instrumente jedoch unerlässlich. Durch Kapillarkräfte werden Kontaminationen wie Blut, Sekrete, Gewebefragmente etc. in einen Zwischenraum zwischen der Seele und der Hülle eingesogen. Ein bei manchen Operationen an der Operationsstelle herrschender Überdruck verstärkt das Eindringen der Kontaminationen in den Zwischenraum zwischen der Seele und der Hülle des chirurgischen Instruments. Zur Reinigung sind beim bekannten chirurgischen Instrument die Hülle und die Seele einfach vom Zangengriff lösbar. Die Hülle ist mit einer Überwurfmutter am Zangengriff befestigt, die die Seele bildende Stange ist eingehängt und durch die Hülle gesichert, so lange die Hülle am Zangengriff befestigt ist.

Die den Stand der Technik vertretende Patentanmeldung US 2001/00 56 283 A1 offenbart ein chirurgisches Instrument mit einem Rohr als Hülle und einem Stab als Seele in der Hülle, die zu einer Betätigung des chirurgischen Instruments mit einem Scherengriff, der an einem Ende des Instruments angeordnet ist, relativ zur Hülle verschiebbar ist. Zum Spülen ist ein Spülanschluss radial von außen an der Hülle angesetzt. Der Spülanschluss weist Abstand vom Ende der Hülle auf, so dass ein Totraum in der Hülle besteht, der nicht gespült wird.

Vergleichbare chirurgische Instrumente offenbaren die internationale Patentanmeldung WO 2005/009 255 A1 offenbar. Auch dort ist ein Spülanschluss mit Abstand von einem Ende an einer Hülle des Instruments angesetzt, so dass ein Totraum in der Hülle verbleibt, der nicht gespült wird. Aufgabe der Erfindung ist, ein gattungsgemäßes chirurgisches Instrument vorzuschlagen, das sich ohne Zerlegen innerlich vollständig reinigen lässt, wobei "innerlich" das Innere der Hülle meint.

Diese Aufgabe wird erfindungsgemäß durch die Merkmale des Anspruchs 1 gelöst. Das erfindungsgemäße chirurgische Instrument weist einen Spülanschluss am distalen Ende der Hülle auf, wobei der Spülanschluss an der Seele angeordnet ist, so dass eine Spülflüssigkeit durch den Spülanschluss am distalen Ende in die Hülle eintritt. Ein Vorteil der Erfindung ist, dass das chirurgische Instrument zur Reinigung nicht zerlegt werden muss, sondern lediglich eine Spülleitung am Spülanschluss angebracht werden muss. Es ist kein Fachpersonal notwendig, das Instrument kann von ungelernten Personen gespült und gereinigt werden. Ein weiterer Vorteil ist, dass beim Reinigen nicht die Gefahr einer Beschädigung oder eines falschen Wiederzusammenbaus des Instruments besteht, weil das Instrument zur Reinigung nicht zerlegt werden muss. Das Innere der Hülle wird vom distalen Ende beginnend über die gesamte Länge der Hülle gespült, die Erfindung vermeidet einen Totraum im distalen Ende der Hülle, der nicht zwangsweise von Spülflüssigkeit durchströmt wird, die durch den Spülanschluss zugeführt wird. Mit Totraum ist ein Bereich der Hülle gemeint, der zwar kontaminiert sein und in den auch Spülflüssigkeit gelangen kann, in dem sich jedoch nicht zwingend eine Strömung ausbildet, weil die Spülflüssigkeit nur von einer Seite in den Totraum eintreten kann, auf der sie auch wieder austreten muss. Die Erfindung hat somit als weiteren Vorteil, dass das Innere der Hülle einfach und von ungelerntem Personal zuverlässig und gründlich gereinigt werden kann und Kontaminationen vollständig ausgespült werden.

Zur Betätigung des chirurgischen Instruments ist ein Scherengriff oder ein Zangengriff vorgesehen, wobei die Aufzählung nicht abschließend ist. Ein Scherengriff weist Hebel mit insbesondere Fingerösen auf, die an ihren Enden vorzugsweise mit Fingern gegriffen werden. Ein Zangengriff wird mit der Hand umgriffen. Die Griffe können in Verlängerung der Hülle, auch parallel oder winklig versetzt (gekröpft) oder in einem Winkel zur Hülle angeordnet sein. Ein Zangengriff kann auch vom distalen Ende der Hülle proximal anstatt distal gerichtet sein. Andere Ausgestaltungen sind nicht ausgeschlossen. Es können zwei bewegliche Griffe oder dgl. oder ein beweglicher Griff oder dgl., der gegen einen feststehenden Griff oder dgl. bewegt wird, vorgesehen sein.

Der Spülanschluss ist in bevorzugter Ausgestaltung der Erfindung ohne Zerlegen des Instruments, also auch ohne Abnehmen eines Scheren- oder Zangengriffs oder einer sonstigen Betätigungs-Mechanik oder Betätigungs-Einrichtung zugänglich, was allerdings nicht zwingend für die Erfindung ist. Wesentlich für die Erfindung ist der Spülanschluss, der ein Spülen des Inneren der Hülle über die gesamte Länge ab dem distalen Ende ohne Totraum sicherstellt. Der Spülanschluss weist insbesondere einen Luer-Anschluss als Steckanschluss oder vorzugsweise als gesicherter, sogenannter Luer-Lock auf.

Die Hülle des erfindungsgemäßen chirurgischen Instruments kann starr, also ein Rohr, oder flexibel sein, ebenso die Seele, die ein Stab, Draht, eventuell auch ein Drahtseil sein oder aus Kunststoff bestehen kann. Longitudinal sind die Hülle und die Seele insbesondere steif zumindest in der Richtung, in der eine Kraft zur Betätigung des Instruments übertragen werden muss, um eine am distalen Ende eingeleitete Verschiebung der Seele gegenüber der Hülle möglichst verlustfrei zum proximalen Ende zu übertragen.

Ein Merkmal der Erfindung ist eine Hohlleitung, die longitudinal fest mit der Seele verbunden ist und an deren distalem Ende der Spülanschluss angeordnet ist. Die Hohlleitung weist eine seitliche Austrittsöffnung für Spülflüssigkeit auf, die in die Hülle des chirurgischen Instruments mündet. Durch den Spülanschluss zugeführte Spülflüssigkeit strömt durch die Hohlleitung und tritt durch deren seitliche Austrittsöffnung in die Hülle aus, durch die die Spülflüssigkeit um die Seele zum proximalen Ende der Hülle fließt, wo sie austritt. Die Hohlleitung kann steif, also ein Rohr, oder flexibel, allerdings longitudinal steif sein. Die Hohlleitung dient der Zuführung der Spülflüssigkeit durch den Spülanschluss in die Hülle des chirurgischen Instruments und der Kraftübertragung auf die Seele zum Betätigen des chirurgischen Instruments. Die Hohlleitung kann auch als Teil der Seele aufgefasst werden.

In bevorzugter Ausgestaltung der Erfindung ist die Austrittsöffnung der Hohlleitung bei unbetätigtem chirurgischen Instrument dicht an einem proximalen Ende eines distalen Endstücks der Hülle angeordnet, also am distalen Ende des Innenraums der Hülle, das Abstand vom distalen Ende der Hülle haben kann. Diese Anordnung der Austrittsöffnung stellt sicher, dass die Spülflüssigkeit am distalen Ende des Innenraums der Hülle in die Hülle eintritt und stellt eine Durchspülung beginnend ab dem distalen inneren Ende der Hülle sicher.

Eine Weiterbildung sieht eine Anzahl longitudinal verteilter Austrittsöffnungen der Hohlleitung vor, wobei die Austrittsöffnungen vorzugsweise auch in Umfangsrichtung der Hohlleitung verteilt angeordnet sind. Die Austrittsöffnungen können gleich- oder ungleichmäßig über die Hohlleitung verteilt sein, die Hohlleitung kann eine Siebhülse sein. Eine Anzahl Austrittsöffnungen vergrößert den Durchtrittsquerschnitt für die Spülflüssigkeit aus der Hohlleitung in die Hülle und stellt einen Austritt der Spülflüssigkeit aus der Hohlleitung in die Hülle an deren innerem, distalen Ende bei unterschiedlichen Schiebestellungen der Seele in der Hülle sicher.

Eine Ausgestaltung der Erfindung sieht einen engen Spalt zwischen der Hülle und der Seele bzw. allgemeiner einen kleinen freien Querschnitt zwischen Hülle und Seele vor. Dadurch wird eine hohe Strömungsgeschwindigkeit beim Spülen und eine infolgedessen eine gute Reinigungswirkung erzielt.

Zu einer guten Reinigung der Außenseite des chirurgischen Instruments sieht eine Ausgestaltung der Erfindung einen Handgriff vor, der über seine Fläche gleich- oder ungleichmäßig verteilt angeordnete Löcher aufweist, die in Bezug auf das Instrument von außen nach innen durchgehen. Mit Fläche ist einen Außen-oder Grifffläche des Handgriffs gemeint. Die Löcher im Handgriff ermöglichen einen Durchtritt von Reinigungsflüssigkeit, die von außen auf das Instrument gesprüht wird, so dass die Reinigungsflüssigkeit durch den Handgriff auf eine Betätigungsmechanik, die sich in einem Zwischenraum zwischen dem Handgriff und dem Instrument befindet, und auf die Außenseite des chirurgischen Instruments gelangt. Die Löcher im Handgriff verringern eine Abschirmung des chirurgischen Instruments und einer etwaigen Betätigungsmechanik gegen von außen gesprühte Reinigungsflüssigkeit durch den Handgriff. Der Handgriff dient zum Halten und/oder zur Betätigung des chirurgischen Instruments, er kann feststehend oder beweglich, beispielsweise ein Scheren- oder Zangengriff sein. Diese Ausgestaltung der Erfindung verbessert die Gesamtreinigung des chirurgischen Instruments innen und außen. Sie kann bei einem gattungsgemäßen chirurgischen Instrument allerdings auch unabhängig von dem Spülanschluss am distalen Ende der Hülle vorgesehen sein.

Eine Ausgestaltung der Erfindung sieht eine Abdichtung am distalen Ende zwischen der Hülle und der Seele auf einem kleineren Umfang als dem Innenumfang der Hülle vor. Bei kreisförmigem Querschnitt ist der Umfang der Durchmesser. Zu diesem Zweck kann die Seele am distalen Ende einen kleineren Durchmesser aufweisen oder die Hohlleitung zur Zuführung der Spülflüssigkeit am distalen Ende der Seele weist einen kleineren Umfang als die Seele auf. Mit der Abdichtung auf kleinerem Umfang lässt sich eine kleinere Reibung und infolge dessen eine niedrigere Betätigungskraft erzielen. Diese Ausgestaltung der Erfindung kann auch unabhängig vom Spülanschluss am distalen Ende der Hülle eines gattungsgemäßen chirurgischen Instruments vorgesehen sein.

Eine Ausgestaltung der Erfindung sieht eine Überlastsicherung vor. Überlastsicherung bedeutet, dass eine Kraft auf die Hülle und die Seele begrenzt wird, wenn deren Verschiebung gegeneinander vor Erreichen einer Endlage blockiert wird, wenn sich also beispielsweise ein Maul am proximalen Ende des chirurgischen Instruments nicht schließen lässt. Zur Überlastsicherung sieht eine Ausgestaltung der Erfindung ein elastisches Element in einem Kraftfluss zur bzw. bei Betätigung des Instruments vor, also eine Elastizität in der Kraftübertragung von einem Scheren- oder Zangengriff oder einer sonstigen Betätigungsmechanik oder Betätigungseinrichtung auf die Hülle und die Seele vor. Wird die Seele am proximalen Ende gegen eine Verschiebung gegenüber der Hülle blockiert, ist der Anstieg der Kraft, die auf die Hülle und die Seele wirkt, bei einem weiteren Schließen des Scheren- oder Zangengriffs oder einer anderen Betätigungsmechanik oder Betätigungseinrichtung geringer als ohne elastisches Element bzw. ohne Überlastsicherung. Das elastische Element kann eine Silikonhülse, eine sonstige elastische Kunststoff- oder Gummihülse, eine Feder, insbesondere eine Schraubenfeder, eine Tellerfeder oder ein Tellerfederpaket sein. Die Aufzählung ist nicht abschließend.

Eine Ausgestaltung der Erfindung sieht vor, dass das elastische Element den Spülanschluss umschließt und gegen ein Widerlager des Spülanschlusses wirkt, also beispielsweise an einer Ringschulter oder einem Flansch des Spülanschlusses anliegt. Die Überlastsicherung lässt sich dadurch raumsparend unterbringen, sie verlängert das chirurgische Instrument allenfalls wenig und vergrößert seinen Durchmesser nicht.

Die Erfindung wird nachfolgend anhand eines in der Zeichnung dargestellten Ausführungsbeispiels näher erläutert. Es zeigen:
- Figur 1: ein chirurgisches Instrument gemäß der Erfindung im Achsschnitt; und
- Figur 2: das chirurgische Instrument in Ansicht in einer um 90° um seine Längsachse gedrehten Darstellung.

Als Ausführungsbeispiel eines erfindungsgemäßen chirurgischen Instruments 1 ist eine chirurgische Schere gewählt worden, wobei auch andere chirurgische Instrumente, beispielsweise eine chirurgische Zange (nicht dargestellt) möglich sind. Das chirurgische Instrument 1 weist ein Rohr 2 als Hülle 3 und einen Drahtstab 4 als Seele 5 auf, die in der Hülle 3 verschiebbar ist. An einem distalen Ende ist die Seele 5 starr beispielsweise durch Schweißen mit einer Siebhülse 6 verbunden, also einem vielfach gelochten Rohr 2, das auch als Hohlleitung 7 aufgefasst werden kann. Löcher sind über den Umfang und longitudinal über die Hohlleitung 7 verteilt und bilden Austrittsöffnungen 8. Ein distaler Abschnitt der Hohlleitung 7 ist lochfrei. Die Hohlleitung 7 weist einen kleineren Durchmesser als die Seele 5 auf, sie verlängert die Seele 5 am distalen Ende koaxial.

Ein distales Ende der Hohlleitung 7 steht aus einem distalen Ende der Hülle 3 vor und es ist ein Spülanschluss 9 am distalen, aus der Hülle 3 vorstehenden Ende der Hohlleitung 7 angebracht. Im Ausführungsbeispiel ist der Spülanschluss 9 ein Luer-Lock, was allerdings nicht zwingend für die Erfindung ist. Spülflüssigkeit, die durch den Spülanschluss 9 zugeführt wird, strömt durch die Hohlleitung 7 und tritt durch deren Austrittsöffnungen 8 in die Hülle 3 aus. Von dort strömt die Spülflüssigkeit zwischen der Seele 5 und der Hülle 3 zum proximalen Ende, wo sie austritt. Da die Seele 5 die Hülle 3 weitgehend ausfüllt, bleibt nur ein kleiner Spalt bzw. freier Querschnitt, durch den die Spülflüssigkeit fließen muss. Die Spülflüssigkeit strömt deswegen mit hoher Geschwindigkeit und hat eine gute Reinigungswirkung.

Am distalen Ende der Hülle 3 ist ein distales Endstück 10 beispielsweise durch Schweißen fest an der Hülle 3 angebracht. Das Endstück 10 ist zylindrisch und weist den gleichen Außendurchmesser wie die Hülle 3 auf. Es ist mit einem zylindrischen, axialen Durchgangsloch versehen, durch das die Hohlleitung 7 axial verschiebbar und abgedichtet durchgeht. Die Austrittsöffnungen 8 der Hohlleitung 7 reichen bis in das distale Endstück 10 der Hülle 3 hinein, so dass sich immer Austrittsöffnungen 8 der Hohlleitung 7 an einem distalen inneren Ende der Hülle 3 befinden, auch wenn die Hohlleitung 7 mit der Seele 5 in der Hülle 3 verschoben ist. Das stellt einen Austritt der Spülflüssigkeit aus der Hohlleitung 7 in die Hülle 3 am distalen inneren Ende der Hülle 3 sicher, so dass Kontaminationen wie Blut, Sekrete und Gewebefragmente zuverlässig auf der gesamten Länge der Hülle 3 beginnend unmittelbar am inneren distalen Ende ausgespült werden. Eine gründliche und vollständige Reinigung des Innenraums der Hülle 3 des chirurgischen Instruments 1 ist dadurch sichergestellt. Das distale innere Ende der Hülle 3 befindet sich am proximalen Ende des distalen Endstücks 10.

Zur Abdichtung zwischen dem distalen Endstück 10 der Hülle 3 und der Hohlleitung 7 kann ein nicht dargestellter Dichtring in eine umlaufende Nut im axialen Durchgangsloch des distalen Endstücks 10 vorgesehen sein. Die Abdichtung erfolgt am lochfreien distalen Abschnitt der Hohlleitung 7, der in das distale Endstück 10 hineinreicht. Die Abdichtung der Hohlleitung 7 im distalen Endstück 10 erfolgt auf dem Durchmesser der Hohlleitung 7, also auf einem kleineren Durchmesser als dem Durchmesser der Seele 5 und dem Innendurchmesser der Hülle 3. Die Abdichtung auf kleinem Durchmesser verringert die Reibung beim Verschieben der Hohlleitung 7 in axialer Richtung.

Zur Betätigung, also zu einer Verschiebung der Seele 5 im bzw. gegenüber der Hülle 3 weist das chirurgische Instrument 1 einen Zangengriff 11 auf. Der Zangengriff 11 weist zwei zylinderschalenförmige Handgriffe 12 auf, die einander gegenüberliegend beiderseits der Hülle 3 angeordnet sind. Die Handgriffe 12 sind longitudinal zur Hülle 3 und in einem spitzen Winkel in Richtung des proximalen Endes schräg nach außen gerichtet angeordnet. Distale Enden der Handgriffe 12 sind an proximalen Enden von Federelementen 13 befestigt, die ähnlich wie Blattfedern ausgebildet sind. Zur Ausbildung der Federelemente 13 ist ein Rohr 27 aus einem elastischen Material auf zwei gegenüberliegenden Seiten in Längsrichtung über einen Großteil seiner Länge geschlitzt, wobei die Schlitze an einem Ende offen sind. Durch die Schlitze sind Halbzylinderschalen gebildet, die etwas auseinander gebogen sind, so dass sie in proximaler Richtung schräg nach aussen stehen. Die Halbzylinderschalen sind über einen Teil ihrer Länge seitlich ausgenommen, d. h. verschmälert. Die Halbzylinderschalen bilden die Federelemente 13, die die Handgriffe 12 halten und führen. Ein schlitzfreier, in Umfangsrichtung geschlossener, distaler Abschnitt 28 des Rohrs 27 umschließt das distale Ende der Hülle 3 und ist auf der Hülle 3 befestigt.

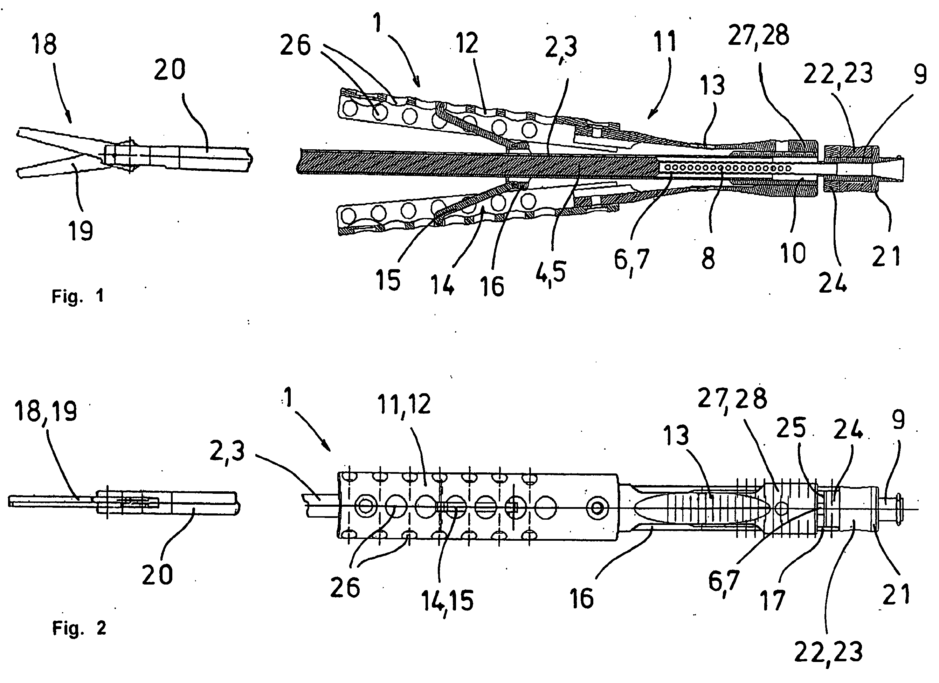

Der Zangengriff 11 weist eine Hebelmechanik 14 auf, die eine Bewegung der Handgriffe 12 quer zur Hülle 3 beim Zusammendrücken des Zangengriffs 11 in eine longitudinale Bewegung zum Verschieben der Seele 5 im bzw. gegenüber der Hülle 3 wandelt. Die Hebelmechanik 14 weist für jeden Handgriff 12 einen am Handgriff 12 angelenkten Hebel 15 auf. Die Hebel 15 verlaufen von den Handgriffen 12 schräg in distaler Richtung nach innen zur Hülle 3, wo sie an einem proximalen Ende einer Schiebehülse 16 angelenkt sind. Die Schiebehülse 16 ist auf einem großen Teil ihrer Länge seitlich ausgeschnitten, weswegen sie in Figur 1 unterbrochen erscheint. Ein distales Ende 17 der Schiebehülse 16 überträgt die Verschiebebewegung auf den Spülanschluss 9 und über diesen und die Hohlleitung 7 auf die Seele 5.

Die Betätigung des chirurgischen Instruments 1 mit dem Zangengriff 11 ist nicht zwingend, es sind auch andere Betätigungsmechanismen oder allgemein Betätigungseinrichtungen zum Verschieben der Seele 5 in bzw. gegenüber der Hülle 3 möglich. Es kann beispielsweise ein beweglicher Handgriff gegen einen feststehenden Handgriff bewegt werden, die Handgriffe können umgekehrt wie gezeichnet angeordnet mit den Gelenken an den proximalen Enden der Handgriffe und die Handgriffe distal über das distale Ende der Hülle 3 überstehend angeordnet sein (nicht dargestellt). Auch kann der Zangengriff 11 quer oder in einem Winkel zur Hülle 3 angeordnet sein oder das chirurgische Instrument 1 weist einen Scherengriff auf (nicht dargestellt). Die Aufzählung ist nicht abschließend sondern spricht lediglich einige Möglichkeiten einer Betätigungseinrichtung oder Betätigungsmechanik für das chirurgische Instrument 1 an.

Am proximalen Ende weist das chirurgische Instrument 1 im Ausführungsbeispiel eine Schere 18 auf, weswegen das chirurgische Instrument 1 auch als chirurgische Schere bezeichnet werden kann. Die Schere 18 weist zwei Klingen 19 auf, die schwenkbar in einem proximalen, gegabelten Endstück 20 der Hülle 3 gelagert sind. Die Klingen 19 sind an der Seele 5 angelenkt und werden durch Verschiebung der Seele 5 im bzw. gegenüber der Hülle 3 verschwenkt. Denkbar ist auch eine Schere 18 mit einer feststehenden und einer schwenkbaren Klinge (nicht dargestellt). Anstelle der Schere 18 kann das chirurgische Instrument 1 beispielsweise auch eine Zange mit zwei Backen anstelle der Klingen 19 aufweisen (nicht dargestellt). Auch diese Aufzählung ist nicht abschließend.

Der Spülanschluss 9 weist einen Flansch auf, der ein Widerlager 21 bildet. An einer proximalen Seite des Widerlagers 21 ist ein elastisches Element 22 auf dem Spülanschluss 9 angeordnet, im Ausführungsbeispiel ist das elastische Element 22 eine Silikonhülse oder eine sonstige elastische Hülse aus Kunststoff oder Gummi. Das distale Ende 17 der Schiebehülse 16 liegt am proximalen Ende des elastischen Elements 22 an, das distale Ende 17 der Schiebehülse 16 bzw. die Schiebehülse 16 insgesamt ist auf dem Spülanschluss 9 verschiebbar. Beim Zusammendrücken der Handgriffe 12 des Zangengriffs 11 verschieben die Hebel 15 die Schiebehülse 16 distal gegenüber der Hülle 3. Die distale Verschiebung der Schiebehülse 16 wird durch die Anlage ihres distalen Endes 17 am elastischen Element 22 über dieses auf das Widerlager 21 des Spülanschluss 9 übertragen. Der Spülanschluss 9 zieht die Hohlleitung 7 distal und verschiebt dadurch die Seele 5 ebenfalls distal in der Hülle 3. Dabei wird die Schere 18 geschlossen. Lässt sich die Schere 18 wegen eines Widerstandes nicht (vollständig) schließen, federt beim Zusammendrucken der Handgriffe 12 das elastische Element 22, es wird elastisch gestaucht. Das Federn des elastischen Elements 22 begrenzt einen Kraftanstieg, der eine Beschädigung der Schere 18 und des chirurgischen Instruments 1 insgesamt verhindert, wenn sich die Schere 18 nicht (vollständig) schließen lässt. Das elastische Element 22 kann deswegen als Überlastsicherung 23 aufgefasst werden.

Im distalen Ende 17 der Schiebehülse 16 ist ein Ring 24 starr befestigt, der auf dem Spülanschluss 9 verschiebbar ist. Am proximalen Ende weist der Spülanschluss 9 einen Flansch als Anschlag 25 auf, an dem der Lochring 24 anliegt. Über den Lochring 24 und den Anschlag 25 wird beim Öffnen der Schere 18 eine Bewegung in proximaler Richtung von der Schiebehülse 16 auf den Spülanschluss 9 und über diesen auf die Hohlleitung 7 und die Seele 5 und umgekehrt übertragen. Geöffnet wird das chirurgische Instrument 1 durch eine Federkraft der Federelemente 13.

Die Handgriffe 12 des Zangengriffs 11 schirmen die Hülle 3 wie Schilde nach außen ab. Die Handgriffe 12 sind mit Löchern 26 versehen, die über die Flächen der Handgriffe 12 verteilt angeordnet sind. Durch die Löcher 26 der Handgriffe 12 kann von außen auf das chirurgische Instrument 1 gesprühte Reinigungsflüssigkeit durchtreten und gelangt an die Hebelmechanik 14, die auf den Innenseiten der Handgriffe 12 angeordnet ist. Die Löcher 26 in den Handgriffen 12 ermöglichen bzw. verbessern eine Reinigung der Hülle 3 und der Hebelmechanik 14 von außen. Als Reinigungsflüssigkeit zum Sprühen von außen kann die gleiche Flüssigkeit wie als Spülflüssigkeit zum Spülen im Innern der Hülle 3 verwendet werden. Verschiedene Wörter für die Flüssigkeit werden wegen ihres Verwendungsortes und der Art ihrer Ein- bzw. Aufbringung verwendet, nicht jedoch weil es sich um unterschiedliche Flüssigkeiten handeln muss.

## Patentansprüche

1. Chirurgisches Instrument mit einer Hülle (3) und einer in der Hülle (3) angeordneten Seele (5), die gegenüber der Hülle (3) verschiebbar ist, wobei das chirurgische Instrument (1) einen Spülanschluss (9) an einem mit Bezug auf den Patienten distalen Ende einer Hohlleitung (7) aufweist, die axial fest mit der Seele (5) verbunden ist und die eine seitliche Austrittsöffnung (8) für Spülflüssigkeit aufweist, die in die Hülle (3) mündet.

2. Chirurgisches Instrument nach Anspruch 1, **dadurch gekennzeichnet, dass** der Spülanschluss (9) bei nicht zerlegtem Instrument (1) zugänglich ist.

3. Chirurgisches Instrument nach Anspruch 2, **dadurch gekennzeichnet, dass** sich die Austrittsöffnung (8) bei unbetätigtem Instrument (1) dicht an einem mit Bezug auf den Patienten distalen inneren Ende der Hülle (3) befindet.

4. Chirurgisches Instrument nach Anspruch 2 oder 3, **dadurch gekennzeichnet, dass** die Hohlleitung (7) eine Anzahl longitudinal verteilter Austrittsöffnungen (8) aufweist.

5. Chirurgisches Instrument nach einem der vorhergehenden Ansprüche, **dadurch gekennzeichnet, dass** ein Spalt zwischen der Hülle (3) und der Seele (5) klein im Verhältnis zu einem Querschnitt ist.

6. Chirurgisches Instrument nach einem der vorhergehenden Ansprüche, **dadurch gekennzeichnet, dass** das Instrument (1) eine Abdichtung der Seele (5) in der Hülle (3) am distalen Ende aufweist, die auf einem kleineren Umfang als dem Innenumfang der Hülle (3) abdichtet.

## Claims

1. Surgical instrument with a covering (3) and a core (5), the core (5) being arranged within the covering (3) and being shiftable with regard to the covering (3), wherein the surgical instrument (1) comprises a flushing connector (9) at a, with regard to the patient, distal end of a hollow pipe (7), where the hollow pipe (7) is axially fixed permanently at the core (5) and has a laterally arranged outlet (8) for a drilling fluid, the outlet (8) leading into the covering (3).

2. Surgical instrument as claimed in claim 1, **characterized in that** the flushing connector (9) is approachable in not disassembled condition of the surgical instrument (1).

3. Surgical instrument as claimed in claim 2, **characterized in that** the outlet (8) is arranged closely at a, with regard to the patient, distal inner end of the covering (3), if the surgical instrument (1) is inoperative.

4. Surgical instrument as claimed in claim 2 or 3, **characterized in that** the hollow pipe (7) comprises a number of outlets (8), that are distributed longitudinally.

5. Surgical instrument as claimed in one of the previous claims, **characterized in that** a gap arranged between the covering (3) and the core (5) is small in relation to a cross section.

6. Surgical instrument as claimed in one of the previous claims, **characterized in that** the surgical instrument (1) comprises at its distal end, a sealing of the core (5) in the covering (3), that proofs at a smaller circumference with regard to the inner circumference of the covering (3).

## Revendications

1. Instrument chirurgical, comprenant une gaine (3) et une âme (5) qui est disposée dans la gaine (3) et qui peut être déplacée en translation par rapport à la gaine (3), sachant que l'instrument chirurgical (1) présente un raccord de rinçage (9) à une extrémité distale, par rapport au patient, d'une conduite creuse (7) qui est axialement fixement reliée à l'âme (5) et qui présente une ouverture de sortie latérale (8) pour un liquide de rinçage, ouverture qui débouche dans la gaine (3).

2. Instrument chirurgical selon la revendication 1, **caractérisé en ce que** le raccord de rinçage (9) est accessible lorsque l'instrument (1) n'est pas désassemblé.

3. Instrument chirurgical selon la revendication 2, **caractérisé en ce que** l'ouverture de sortie (8) se trouve, lorsque l'instrument (1) n'est pas actionné, tout près d'une extrémité intérieure, distale par rapport au patient, de la gaine (3).

4. Instrument chirurgical selon la revendication 2 ou 3, **caractérisé en ce que** la conduite creuse (7) présente un certain nombre d'ouvertures de sortie (8) longitudinalement réparties.

5. Instrument chirurgical selon l'une des revendications précédentes, **caractérisé en ce qu'**un interstice entre la gaine (3) et l'âme (5) est de petite taille par rapport à une section transversale.

6. Instrument chirurgical selon l'une des revendications précédentes, **caractérisé en ce que** l'instrument (1) présente à l'extrémité distale un joint d'étanchéité de l'âme (5) dans la gaine (3), joint qui assure l'étanchéité sur une plus petite circonférence que la circonférence intérieure de la gaine (3).
